# EUROPEAN PATENT APPLICATION

(11) **EP 4 420 677 A1**
(43) Date of publication of application: **28.08.2024**
(21) Application number: 23305238.0
(22) Date of filing: 22.02.2023
(51) Int. Cl.: A61K 39/00, A61K 39/39, A61P 35/00, C12N 9/12

(54) **VACCINES AGAINST TERT-POSITIVE TUMORS**

(71) Applicant: Altevax, 75116 Paris (FR); Assistance Publique Hôpitaux de Paris, 75012 Paris (FR); Université Paris Cité, 75006 Paris (FR)
(72) Inventor: CARPENTIER, Antoine, 75007 PARIS (FR); BANISSI, Claire, 94300 VINCENNES (FR)
(74) Representative: Flesselles, Bruno F.G.

(57) **Abstract**

The invention relates to a new peptide containing a TERT epitope which presents good immunogenicity and is useful for immunotherapy of cancer.

## Description

The invention pertains to the field of cancer treatment, and to the identification, modification and formulation of a specific peptide useful for immunotherapy of cancer.

Telomeres are specialized structures at the end of eukaryotic chromosomes. As standard DNA polymerases cannot fully replicate linear DNA, a loss of 50 to 100 nucleotides occurs with each cell division, leaving non-replicated DNA at the 3'end (Zhao 2009). After a certain number of cell doublings, telomeres shortening reaches a critical length threshold and the cells undergo a growth arrest (Colebatch 2019). In cells requiring a large number of divisions, such as stem cells, this so-called "end-replication" problem can be solved by the expression of a catalytic protein named telomerase, which prevents the telomeres from shortening (Hayflick 1998). Telomerase constitutes a complex system of large molecules among which the telomerase reverse transcriptase (TERT) plays a critical role (Huang et al., 2013). Besides its telomeric function occurring within the nucleus, extra-telomeric (also called non-canonical) functions of TERT have been reported and are mainly exhibited by mitochondrial TERT. Mitochondrial TERT protects mitochondrial DNA through binding to subunits of complex I in the respiratory chains and decreases the level of reactive oxygen species (ROS) (Rosen 2020). The non-canonical extra-telomeric functions of telomerase can be misused by cancer cells to promote oncogenesis. For instance, TERT can stimulate cell proliferation by inducing the expression of several growth-promoting genes (such as EGFR), or increase cell migration (Liu 2016), or increase mitochondrial membrane potential, inducing resistance to chemotherapeutic agents and pro-apoptotic stimuli (Chiodi and Mondello 2012). TERT can also stimulates angiogenesis and impact the tumor micro-environment made by interfering with the Wnt/β-catenin signaling pathway and positively regulates the NF-κB pathway, a key transcription factor which activates many inflammatory genes (Ghosh 2012; Li & Tergaonkar, 2014).

TERT expression is tightly regulated in normal cells (Armstrong 2000). Multiple mechanisms contribute to this regulation, including epigenetic, transcriptional, and post-transcriptional processes. The epigenetic regulation is mediated by TERT promoter methylation status. (Lee 2018). The TERT promoter contains binding motifs for several transcription factors, notably the MAX/MAD1 complex that acts as a repressor of TERT expression in physiological conditions (Dogan 2021). Post-transcriptional regulation is mainly conveyed by the alternative splicing of hTERT pre-mRNA (Slusher 2020). To date, 22 hTERT alternative splicing variants have been reported, but only the full-length hTERT mRNA without any deletion or insertion exhibits telomerase activity (Wong 2014).

Under physiological conditions, TERT is undetectable or only present at low levels in most healthy tissues but remains constitutively expressed in stem cells, in early precursor cells of the bone marrow, in spermatocytes, the thymus, the spleen, and a certain subset of lymphocytes within the lymph nodes (Hiyama 2007).

In cancer, Telomerase reactivation is reported in approximately 85 to 95% of human primary tumors (Low 2013). A pan-cancer study revealed that somatic TERT promoter mutations is particularly frequent in glioma (85%), cutaneous melanoma (85%), urothelial carcinoma (73%), poorly differentiated (21 - 60%) and anaplastic thyroid cancers (13 - 73%), or hepatocellular carcinoma (44%) (Gupta 2021).

Aberrant upregulation of TERT in cancer cells can be driven by both genetic and epigenetic mechanisms including alternative RNA splicing (increasing full-length hTERT mRNA that allows telomerase activity), TERT promoter hypermethylation and, mainly, TERT promoter mutations. The two most frequent promoter mutations, C228T and C250T, are found in two hotspot positions, corresponding respectively to -124 bp (C > T) and -146 bp (C > T) position upstream from the ATG start site. These C228T and C250T mutations accounted for 77% and 21% of TERT alterations, respectively (Killela 2013). TERT promoter mutations are one of the most common genetic alterations in adult gliomas, occurring in more than 95% of the oligodendrogliomas and in 80% of IDH-wildtype glioblastoma (Pierini et al. 2020).

Due to its pivotal biological role in cancer, TERT thus represents a promising target for immunotherapy,
More than 25 immunogenic TERT epitopes have been identified, some being associated with MHC class I-mediated enhancement of CD8+ T-cell response, other being implicated in MHC class Il-mediated induction of CD4+T-cell response (see in particular Vonderheide, Biochimie 90 (2008) 173e180 , table 1, page 175 ; Ellingsen 2021; WO2018206462; Dosset et al (Cancers 2020, 12(6), 1687). Some MHC class II peptides were identified with the capacity to bind not only the most commonly expressed HLA DR molecules but most HLA class II (Godet et al. Clin Can Res 2012; EP2639299). The "universal" TERT peptides UCP2 (SEQ ID NO: 1) and UCP4 (SEQ ID NO: 2) are immunogenic in a large number of patients (Dosset et al Clin Can Res 2012, and Immunooncology 2013), including lung cancer patients (Adotevi et al 2023) and are currently under evaluation in newly-diagnosed glioblastoma (NCT04280848).

**Table 1. List of identified immunogenic TERT epitopes (extracted from Dosset et al and Vonderheide (op. cit.)**

| **Peptide/ Epitope** | **Sequence** | **SEQ ID** | **Main HLA Restriction** |
|---|---|---|---|
| p68 | APSFRQVSCLKELVA | 8 | HLA-DR |
| p911 | DEALGGTAFVQMPAH | 9 | HLA-DP4 |
| UCP1 | PAAFRALVAQCLVCV | 10 | HLA-DR |
| UCP2 | KSVWSKLQSIGIRQH | 1 | HLA-DR |
| UCP3 | GTAFVQMPAHGLFPW | 11 | HLA-DR |
| UCP4 | SLCYSILKAKNAGMS | 12 | HLA-DR |
| p541 | LAKFLHWLMSVYVVE | 13 | HLA-DP4 |
| p573 | LFFYRKSVWSKLQSI | 14 | HLA-DP4 |
| p613 | RPALLTSRLRFIPKP | 15 | HLA-DP4 |
| p386 | YWQMRPLFLELLGNH | 16 | HLA-DP4 |
| p660 | ALFSVLNYERARRPGLL GASVLGLDDIHRA | 17 | HLA-DR |
| p663 | SVLNYERARRPGLLG | 18 | HLA-DR |
| p673 | PGLLGASVLGLDDIH | 19 | HLA-DR |
| GV1001 | EARPALLTSRLRFIPK | 20 | Promiscuous DR, DP, DQ |
| p766 | LTDLQPYMRQFVAHL | 21 | DR4, DR11, DR15 |
| p672 | RPGLLGASVLGLDDI | 22 | DR1, DR7, DR15 |
| l540 | ILAKFLHWL | 23 | HLA-A2 |
| R865 | RLVDDFLLV | 24 | HLA-A2 |
| 572Ya | YLFFYRKSV | 25 | HLA-A2 |
| 988Ya | YLQVNSLQTV | 26 | HLA-A2 |
| R38a | RLGPQGWRV | 27 | HLA-A2 |
| K973 | KLFGVLRLK | 28 | HLA-A3 |
| V324 | VYAETKHFL | 29 | HLA-A24 |
| V461 | VYGFVRACL | 30 | HLA-A24 |
| Y325 | YAETKHFLY | 31 | HLA-A1 |
| M1 | MPRAPRCRA | 32 | HLA-B7 |
| A4 | APRCRAVRSL | 33 | HLA-B7 |
| A68 | APSFRQVSCL | 34 | HLA-B7 |
| R277 | RPAEEATSL | 35 | HLA-B7 |
| R342 | RPSFLLSSL | 36 | HLA-B7 |
| R351 | RPSLTGARRL | 37 | HLA-B7 |
| L1123 | LPSDFKTIL | 38 | HLA-B7 |
| T1088 | TYVPLLGSL | 39 | HLA-A24 |
| C845 | CYGDMENKL | 40 | HLA-A24 |
| A167 | AYQVCGPPL | 41 | HLA-A24 |

Multiple other clinical trials with various TERT peptide vaccines have been conducted in various solid tumors and myeloma. Immune responses against TERT were detected in ≥ 50% of the immunized patients, and tumor responses were observed in some cases, without any significant side effects (Ellingsen 2021). One TERT vaccine has advanced to phase III in patients with advanced pancreatic cancer but failed to demonstrate a survival advantage over chemotherapy (Middleton 2014). Collectively, the clinical trials indicate that therapeutic TERT-based vaccination can trigger T cell responses to TERT but has, so far, limited anticancer efficacy when used alone. The effectiveness of TERT vaccination might be significantly improved by improving the vaccine technology and/or with concomitant treatment with immune-checkpoint inhibition, such as anti-PD1 antibodies, which enhances T cell responses in patients with cancer.

Priming lymphocytes or antibodies against a specific target (a process named immunization) result from the presentation of one antigen by antigen-presenting cells to T cells. This process can be achieved *in vitro,* but is more easily achieved *in vivo,* by administration of the antigen into living animals or humans (a procedure named vaccination). Vaccines, despites all the progress made, are still facing several limitations. Most antigens are poorly immunogenic. The dose of a peptidic antigen required to trigger the immunity (usually within the range of 10 to 300µg) might be a limiting factor, especially when antigen is difficult to manufacture, or when demand exceeds production capacity. Moreover, induction of CD8+ lymphocytes remains a difficult challenge because antigen injected extracellularly are usually presented by MHC class II and not by MHC class I (thus preferentially leading to CD4+ lymphocytes and antibodies). Finally, vaccine technologies, such as emulsion, liposomes, nanoparticles, fusion molecules, DNA and RNA vaccines can be either unstable or difficult to synthesize, making the cost of manufacturing sometimes prohibitive.

Adjuvants are thus generally used for increasing the immunogenicity of the antigen administered. WO2017089529 discloses that melanin can be used as an adjuvant to increase immune response against an antigen bearing epitopes.

WO2021165306 discloses that addition of an amino acid with a nucleophilic residue to a peptide is useful to improve its immunogenicity when complexed with melanin.

The inventors modified the epitope UCP2 disclosed in Godet et al. Clin Can Res 2012 & Dosset et al Clin Can Res 2012, by adding a serine at its N-terminus and showed that this modified peptide complexed to melanin increases the immune response generated when administered *in vivo,* when compared to the preferred amino-acid additions disclosed in WO 2021165306 (addition of cysteine, or lysine or methionine at its end-terminus).

In a first embodiment, the invention thus relates to a polypeptide or a peptide comprising SEQ ID NO: 7, or a peptide consisting in SEQ ID NO: 7, to generate an immune response against TERT that is useful for the treatment of cancers.

As indicated below, the peptide comprises preferably at most 100, more preferably at most 50 amino acids. When the peptide is bigger than SEQ ID NO: 7, SEQ ID NO: 7 may be located at the N-terminus of the peptide, or somewhere else within the peptide. The peptide may however contain more than 100 amino acids. It may be a protein, having a biological activity.

In some embodiments, the polypeptide or peptide that comprises SEQ ID NO: 7 further comprises other immunogenic epitopes, in particular CD4 or CD8 epitopes of antigens, in particular of cancer antigens, notably the cancers targeted by the immunogenic compositions herein disclosed. The other epitopes can be linked to SEQ ID NO: 7 by stretches of amino acids (this is the preferred embodiment) or any other acceptable linkers such as polyether compounds or other linkers used in dendrimer constructs. When melanin is used as an adjuvant, it is preferred when SEQ ID NO: 7 is at the N-terminus of the polypeptide or peptide.

The other epitopes may be other epitopes from TERT or epitopes from other proteins than TERT, the expression of which is associated with the same cancers than TERT.

The other epitopes may be universal T helper epitopes such as pan-DR epitope (PADRE) and Pol₇₁₁ epitopes. The literature widely discloses other universal T helper epitopes. This can improve the immune response against cells expressing TERT *via* the response against SEQ ID NO: 7.

The invention also relates to a nucleic acid molecule coding for the polypeptides or peptides disclosed above. In some embodiments, the nucleic acid molecule is DNA. In other embodiments, the nucleic acid molecule is RNA. In some embodiments, the nucleic acid molecule is a chimera DNA-RNA. The nucleic acid may be protected from degradation, when administered to a subject, by methods known in the art, in particular by liposome encapsulation.

The polypeptide or peptide herein disclosed can be used for treating or preventing cancers where TERT expression is high, and notably in glioblastomas, gliomas, melanomas, hepatocellular carcinoma, lung carcinomas, urothelial or thyroid cancers.

The invention thus relates to the polypeptide or peptide herein disclosed, the nucleic acid molecule or the vaccine composition disclosed below for use as a medicament, and in particular as a vaccine (whether prophylactic or therapeutic).

The invention thus also relates to a vaccine composition (or an immunogenic composition) comprising the polypeptide, peptide or the nucleic acid molecule herein disclosed. In the vaccine composition, the polypeptide, peptide or the nucleic acid molecule is formulated with appropriate excipients and optionally some adjuvants to be injected to a mammal, notably a human being. In particular, the administration can be an intramuscular, intravenous, subcutaneous, intraperitoneal injection, or an injection directly into the tumor. In other embodiments, the vaccine composition can take the form of an oral, inhalable or intradermal composition, notably in the form of a patch. Subcutaneous administration is of particular interest.

It is preferred when the vaccine composition comprises a polypeptide or peptide herein disclosed, and an adjuvant, and in particular when the adjuvant is melanin. One shall preferably use a synthetic melanin (*i*.*e*. a melanin obtained *in vitro* from oxidative polymerization of a precursor) melanin. In particular, the melanin is a soluble melanin. In this embodiment, the peptide shall be complexed or bound to the melanin. The vaccine composition can be obtained by oxidative polymerization of a melanin precursor in the presence of the peptide, as disclosed in WO2017089529, or by addition of the peptide to an already synthetized melanin as disclosed in WO2021165306.

The invention also relates to the polypeptide, peptide, the nucleic acid or the vaccine composition herein disclosed for use for the treatment of cancer.

The invention also relates to the use of a polypeptide, peptide, a nucleic acid or an immunogenic composition herein disclosed for the preparation of a medicament for the prevention or treatment of a cancer in a patient. In this embodiment, the medicament contains the polypeptide, peptide, the nucleic acid or the immunogenic composition as well as appropriate excipients or adjuvants.

The vaccine may be a prophylactic (*i*.*e*. intended to protect the recipient against the development of a disease) or a therapeutic (*i*.*e*. intended to help the recipient fight an already present disease) vaccine. The disease is linked to the target antigen (TERT), expressed or presented by cells during the course of the disease.

The invention also relates to a method for treatment of a patient in need thereof (in particular a patient with a cancer), comprising administering an effective amount of the polypeptide, peptide, nucleic acid or immunogenic composition herein disclosed to the patient. Such administration leads to the generation of an immune response directed against TERT, which would then attack and eliminate the tumor cells, thereby providing a therapeutic effect.

The invention also relates to a method for protecting a patient against a cancer, comprising administering a therapeutic or effective amount of a polypeptide, a peptide, a nucleic acid or an immunostimulatory composition as disclosed herein to the patient, so as to induce an immune response against TERT that is associated with the cancer, wherein the immune response has a protective (prophylactic) effect against the cancer.

In particular, the cancer is a low or high grade glial tumor.

In another embodiment, the cancer is a melanoma.

In another embodiment, the cancer is a urothelial carcinoma.

In another embodiment, the cancer is a lung cancer.

In another embodiment, the cancer is a small cell lung cancer

In another embodiment, the cancer is a thyroid cancer.

In another embodiment, the cancer is a hepatocellular carcinoma.

An "effective amount" or a "therapeutic amount" of an agent, as used herein, is the amount sufficient to induce beneficial or desired results, such as clinical results or onset of an immune response, in particular a T-cell mediated immune response. In the present context, a therapeutic amount of an agent is, for example, an amount sufficient to achieve onset of an immune response against the antigen, and reduction in the severity of a symptom of the disease linked to the antigen, as compared to the situation observed without administration of the composition. An effective amount is an amount that provides therapeutic improvement while minimizing side or adverse effect. One can use, as effective amounts, 10 µg to 5 mg of antigen, preferably between 20 µg and 500 µg. In case melanin is used in the vaccine formulation, the amount of melanin that can be used may be comprised between 40 µg and 10 mg, in particular between 40 µg and 1 mg

The resulting vaccine formulation can be used to protect an animal against a disease implicating (i.e. involving and/or concerning) cells that are expressing inside, at their surface, or secreting TERT.

Alternatively, the polypeptide, peptide, nucleic acid or immunostimulatory composition can be used *in vitro* in presence of live cells (for example macrophages, dendritic cells, antigen-presenting cells or lymphocytes), to sensitize them to the antigen, for instance before administration (preferably injection) in humans (priming). The resulting composition will thus elicit an immune response against the antigen TERT in the recipient. In particular, US 6210662 discloses such principle of forming therapeutic or immunogenic compositions consisting of antigen presenting cells activated by contact with an antigen complex.

The invention thus relates to an *in vitro* method for priming or stimulating CD4 or CD8 lymphocytes against TERT, comprising contacting and incubating a polypeptide, a peptide or a nucleic acid molecule or an immunogenic composition herein disclosed, optionally with an adjuvant, with antigen presenting cells and T-lymphocytes. It is preferred when the antigen presenting cells and T-lymphocytes have been isolated from a patient with cancer. The resulting lymphocytes can then be recovered and administered to a patient for the treatment of cancer involving TERT.

The invention also relates to a method to detect an immune response against TERT (in particular against the epitope depicted by SEQ NO: 7) by co-incubation of an immunostimulatory composition as disclosed herein with a patient's tissue containing immune cells (such as blood, or isolated lymphocytes) and detecting a specific immune response of the patient's lymphocytes against the TERT antigen present in the immunostimulatory composition. Such response can be detected by measure of molecules secreted by the lymphocytes, such as cytokines and in particular gamma-interferon.

The invention also relates to a method to detect an immune response against TERT (in particular against the epitope depicted by SEQ ID NO: 7) by co-incubation of a population of antigen presenting cells (such as monocytes, or dendritic cells) with a patient's tissue containing immune cells (such as blood, or isolated lymphocytes) and detecting a specific immune response of the patient's lymphocytes against the TERT antigen present in the immunostimulatory composition. Such response can be detected by measure of molecules secreted by the lymphocytes, such as cytokines and in particular gamma-interferon. The antigen presenting cells have previously been incubated in the presence of the immunogenic composition herein disclosed, and thus present the epitome depicted by SEQ ID NO: 7 at their surface by the MHC I molecules.

These methods are performed *in vitro.*

### Melanin

It is reminded that a "melanin" is a pigment being a macromolecule obtained from the oxidative polymerization of precursors related to indole or cathecol (generally starting with the oxidation of the amino acid tyrosine (or another precursor), followed by polymerization. This oxidation is a critical step and is generally mediated by the enzyme tyrosinase, which will convert tyrosine to DOPA. WO2017089529 and WO2021165306 disclose the process of synthesis of eumelanin. As a melanin usable as an adjuvant with the epitope herein disclosed, on can use a "natural" melanin, as could be found in nature, such as an eumelanin, a MAPs-like polymer (containing a high proportion of a melanin precursor), or a synthetic melanin molecule that is obtained by *in vitro* oxidative polymerization of precursor derivatives such as the ones described below. Synthetic melanin, prepared in particular by oxidation of tyrosine or L-Dopa with hydrogen peroxide, is thus sold as such, for instance by Sigma Aldrich.

A preferred melanin, in the context of the present application, is eumelanin.

### Obtaining a synthetic melanin

A synthetic melanin is obtained after oxidative polymerization of melanin precursors *in vitro.*

Polymerization of melanin precursors can be performed by methods known in the art. In particular, the melanin precursor may be incubated, with or without buffer, with an enzyme such as phenylalanine hydroxylase, tyrosinase, mushroom tyrosinase, tyrosine hydroxylase, peroxidase, phenol-oxidase, Dopachrome tautomerase, DHICA oxidase, DHI oxidase. The choice of the enzyme will be made by the person skilled in the art depending on the nature of the precursor present in solution before polymerization. It is favored when the oxidative polymerization is performed in presence of tyrosinase.

The mixture is also exposed to an oxidizing agent (oxidant or oxidative agent) as disclosed below in order to promote the polymerization and obtain the synthetic melanin.

Among others, the person skilled in the art may optimize various parameters such as the ratio of melanin precursors is a mixture is used, the type of oxidant, pH, buffer, length of incubation, or temperature of reaction.

In particular, melanin synthesis may be influenced by pH (alkaline pH promoting auto-oxidation of catechol), and presence of metal ions (such as Cu²⁺, Ni²⁺, Fe³⁺, Fe²⁺, Co²⁺, Zn²⁺, Mn²⁺, Mg²⁺...) present in the incubation solution (Palumbo et al, Biochim Biophys Acta. 1987; 13;925(2):203-9; Palumbo et al, Biochim Biophys Acta. 1991;1115(1):1-5; WO95009629). Thus, working at a pH 8.5+/-0.5 is adapted. Physico-chemical conditions can be modified to increase the reaction kinetics, such as increasing temperature above 20°C (for example between 60 and 80°C), of bubbling air into the reaction mixture, or increasing the atmospheric pressure.

Such synthetic melanin may be more homogeneous than natural melanin and could thus be distinguished from it. It is preferred, in the context of the present invention, to use a synthetic eumelanin-like, in particular obtained from *in vitro* oxidative polymerization of L-Dopa.

In an embodiment, the synthetic melanin (post polymerization) is purified by filtration on a 5kDa-100kDa filter, preferably a 10kDa filter.

In a preferred embodiment, the synthetic melanin is a soluble melanin, i.e. is in the form of particles of less than 500nm.

When synthetized, melanin is washed by ultrafiltration or by filtration on an approximately 10kDa filter (melanin remains on the retentate), then resuspended in in water or buffer (such as a phosphate buffer). Melanin can be filtered through a 0.2 µm filter for sterility. Thus, in an embodiment, the melanin is resuspended in water with or without buffer (such as phosphate buffer) prior to being mixed with the peptide to obtain an immunogenic composition

### Melanin Precursor

A "melanin precursor" is a molecule that is used or synthetized during the synthesis of a melanin, in particular a eumelanin, *in vitro.* One can cite: L-phenylalanine, L-tyrosine, L-dopa, dopaquinone, cyclodopa, dopachrome, Dihydroxyindole carboxylic acid or 5,6-dihydroxyindole-2carboxylic acid (DHICA), indol 5,6 quinone, 5,6-dihydroxyindole (DHI), dopamine-o-quinone, Dopamine leukodopaminochrome, leukodopachrome (cyclodopa), dopaminochrome, norepinephrine, noradequinone, noradenochrome, epinephrine, epinephrine-o-quinone, adenochrome, 3-amino-tyrosine, 6-hydroxy-Dopa, dihydrocaffeic acid, caffeic acid, methide, benzothiazole, benzothiazine, dihydroesculetin and others.

Indeed, the term "melanin precursor" further includes derivatives of such precursors and/or polymers containing a high proportion of such precursors (such as in Mussel Adhesives Proteins). Such melanin precursors and derivatives are described in WO2017089529 (incorporated by reference with regards to this teaching) and can be used as equivalent melanin precursors in the context of the present invention.

The melanin precursor is preferably selected from the group consisting of DHICA, DHI, L-dopa, L-tyrosine, D-dopa, 6-hydroxy-Dopa, dopaquinone, cyclodopa, dopachrome, dopamine-o-quinone, dopamine, leukodopaminochrome and dopaminochrome.

A preferred melanin precursor is L-dopa. Another preferred melanin precursor is DHICA. Another preferred melanin precursor is DHI. Another preferred melanin precursor is L-tyrosine. In a specific embodiment, the melanin precursor is a mixture of DHICA and DHI. In another embodiment, the melanin precursor is dopachrome.

Other melanin precursors or derivatives thereof are described in the art, such as the products described in WO2017089529.

### Oxidizing agent

An "oxidizing agent" or "oxidizing molecule" is a compound that is able to provide oxygen to a solution containing melanin precursors and promote polymerization thereof and formation of a melanin macromolecule.

Oxidizing agents that can achieve this goal comprise oxygen, hydrogen peroxide, ammonium persulfate, ferric ions, sodium iodide together with hydrogen peroxide, and treatment with a salt of a transition metal cation such as copper sulfate as a catalyst for air oxidation.

It is thus preferred when the oxidizing agent is chosen in the group consisting of oxygen, hydrogen peroxide, ammonium persulfate, and ferric ions.

### Vaccine, immunogenic or immunostimulatory composition

An, "immunogenic or immunostimulatory composition" is a composition that is able to generate an immune response in an animal when administered to said animal. Preferably, said animal is a mammal, but is can also be a bird (such as a chicken, a duck, a goose, a turkey, a quail), in particular when the composition is used in avian livestock. The animal may also be a fish, as the immunogenic composition may be used in fish farming.

The immunogenic composition is preferably used in mammals. Such mammals are preferably human beings, but can also be other mammals, when the composition is used in the veterinary field, in particular for inducing immunity in livestocks such as cattle (cows), sheeps, goats or horses, but also for pets such as dogs or cats.

The immunogenic composition is thus a composition that contains an antigen, in particular a peptide containing epitopes from an antigen, and that is able to generate an immune response against such antigen. The generated immune response can be a cellular (T-cell mediated) or a humoral (B-cell mediated, production of antibodies) immune response. The immunogenic composition may also induce both a cellular and a humoral immune response.

The cellular immune response can be a CD8 T lymphocytes mediated response (ie cytotoxic response), or a CD4 T lymphocytes mediated response (helper response). It can also combine a cytotoxic and helper cellular immune response. The helpher response may involve Th1, Th2 orTh17lymphocytes (such lymphocytes being able to elicit different cytokine responses, as is known in the art).

The immunogenic composition may allow a better presentation of the antigen present therein, through MHC1 or MHC2 pathways.

The immunogenic composition shall contain a polypeptide or a peptide comprising SEQ ID NO: 7. It may also be a mixture of polypeptides or peptides.

In some embodiments, the immunogenic composition contains a nucleic acid coding for a polypeptide or a peptide comprising SEQ ID NO: 7. Administration of this immunogenic composition makes it possible to obtain expression of the polypeptides or peptides within cells *in vivo,* ad obtaining an immune response against these polypeptides or peptides. Cells may also be transfected *in vitro* by the nucleic acid coding for a polypeptide or a peptide comprising SEQ ID NO: 7, and the resulting cell composition can be used as the immunogenic composition.

### Adjuvant

An "adjuvant" is a substance that has the capacity to modify or enhance the immune response to an antigen. In other words, the immune response against the antigen may be higher or different in the presence of the adjuvant than when the adjuvant is not present (that includes when the response is modified, for example when the subset of T cells that are activated in the presence of the adjuvant is different from the subset activated in the absence of the adjuvant). A lot of adjuvants are known in the art and have been widely used in the vaccine field.

One can cite alum, emulsions (either oil-in-water or water-in-oil, such as Freund's Incomplete Adjuvant (IFA) and MF59^{®}), PRR (Pattern recognition receptors) Ligands, TLR3 (Toll-Like Receptor 3) and RLR (RIG-I Like Receptors) ligands such as double-stranded RNA (dsRNA), or synthetic analogs of dsRNA, such as poly(I:C), TLR4 ligands such as bacterial lipopolysaccharides (LPS), MPLA (monophosphoryl lipid A), in particular formulated with alum, TLR5 ligands such as bacterial flagellin, TLR7/8 ligands such as imidazoquinolines (i.e. imiquimod, gardiquimod and R848), TLR9 ligands such as oligodeoxynucleotides containing specific CpG motifs (CpG ODNs) or NOD2 (Nucleotide-binding oligomerization domain-containing protein 2) ligands. The term ligand above describes preferably an agonist of the receptor, *i.e.* a substance that binds to the receptor and activates the receptor, in particular for TLR3 and TLR9 receptors.

Melanin as described in WO2017089529, or in WO2021165306 acts as an adjuvant and its combination with another adjuvant may also be used. When melanin is used and another adjuvant is added, it is preferred when it is selected in the group consisting of TLR3 agonists and TLR9 agonists and in particular when this adjuvant that is further added is chosen among Polyinosinic:polycytidylic acid (poly I:C) and CpG oligonucleotides.

### Peptide

A peptide is a chain of amino acids linked by peptide bonds. In the context of the invention, a peptide shall comprise at least 9 amino acids, more preferably at least 10 amino acids, more preferably at least 11 amino acids, or at least 12 amino acids. In some embodiments, the peptide shall contain at most 100 amino acids, more preferably at most 50 amino acids, more preferably at most 30 amino acids, more preferably at most 25 amino acids. Peptides between 10 and 25 amino acids are well adapted. In other embodiments though, the peptide (which may be referred to as a polypeptide) may contain more than 100 amino acids. It may be a protein.

One or more amino acids of the peptide may be artificial (different from one of the 20 amino acids found in proteins in nature). Such artificial amino acid may be a D-amino acid, or a non-natural amino acid (such as citrulline, hydroxyproline, norleucine 3-nitrotyrosine, nitroarginine, ornithine, naphtylalanine...).

The peptide may be capped or modified at its N and/or C-terminus. In particular, acetylation or capping of the N-terminus helps to minimize amino peptidase degradation of the peptide when amidation of the C terminus helps to stabilize the peptide from carboxypeptidase degradation.

In the context of the invention, a biologically active peptide should thus contain SEQ ID NO: 7. As indicated, this sequence might be included in a large protein or in a longer peptide, and further modified with glycosylation or extremities protection.

### Vaccine

In the context of the invention, a vaccine is a composition that is administered to an animal produce or artificially increase immunity to a particular antigen. It is thus understood that the terms "immunogenic composition", "immunostimulatory composition" and "vaccine" can indifferently be used.

### Obtaining an immunogenic composition

An immunogenic composition can be obtained by combining a polypeptide or a peptide containing the modified epitope herein disclosed, with an adjuvant.

In particular, the polypeptide or peptide bearing the modified epitope is combined to a melanin, in particular a synthetic melanin as herein described.

Polypeptides or peptides can be added to a synthetic melanin solution as disclosed above (weight ratio polypeptide or peptide/melanin between 1/1 and 1/10) and incubated for various periods of time before usage, with the duration that can depend on temperature of incubation. The resulting solution can be washed and resuspended in water or in any appropriate buffer.

The binding of the polypeptide or peptide to the melanin can be verified by Tricine-SDS-PAGE analysis as described in Carpentier (2017). Briefly, samples (peptide-Mel or peptide alone) are loaded on acrylamide gels. Following electrophoresis, the gels are stained with Coomassie Brilliant Blue R-250, allowing the quantification of the free peptide in the gel. The binding of peptides to melanin can be expressed as the ratio: [amount of unbound peptide in samples Peptide-Mel / amount of peptides in control samples containing peptides alone.

The immunostimulatory composition may also comprise another adjuvant as disclosed above. In a preferred embodiment, the adjuvant is added to the composition obtained just before administration, i.e. less than one hour before administration.

### FIGURES

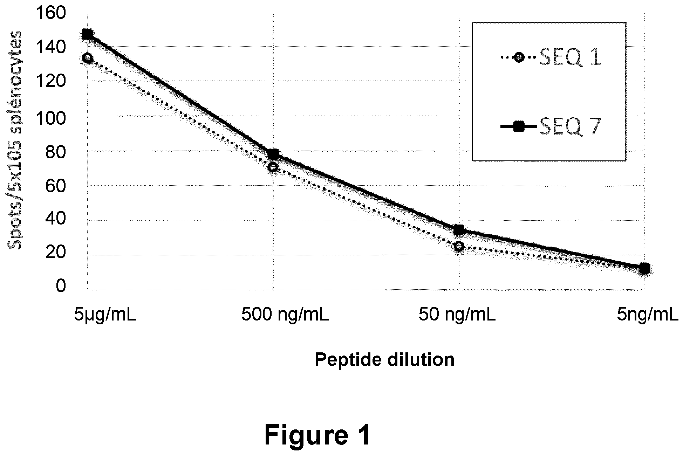

Figure 1: cross reactivity of T-cell responses obtained after immunizations with SEQ 7 in transgenic SURE mice. Mice were immunized against SEQ 7, as described in table 1, and sacrificed on day 8. The splenocytes (5.10⁵ cells /well) were re-stimulated in vitro for 18 hours with either SEQ ID NO: 7 or the native epitope SEQ ID NO: 1 (both non-conjugated to melanin), at various concentrations (5 µg/ml to 5 ng/ml) and the numbers of IFNγ-SFCs (Spot forming cells) were measured after a 18-hour incubation.

### EXAMPLES

For the purpose of selecting an optimized TERT antigen to be used in human patients, several peptides containing one of the known TERT immune epitope (SEQ ID NO: 1) were screened in a melanin-based vaccine, as described in WO2021165306.

These peptides (SEQ ID NO: 4 to SEQ ID NO: 7; table 2) all contain the UCP2 epitope (SEQ ID NO: 1) with an additional amino-acid at the NH2-terminal, as it was disclosed in WO2021165306 that such modifications increase the efficacy of melanin-based vaccine. This application teaches that cysteine, acetylcysteine, methionine, proline, hydroxyproline, histidine and lysine are preferred amino acids that can increase peptide binding, cysteine being the preferred amino-acid for optimal binding.

**Table 2. list of sequences used, and the corresponding T-cell responses obtained after subcutaneous immunizations in transgenic Surel1 mice.**

| | Peptidic sequence | T-cell Immune response: mean +/-SEM (number of mice) |
|---|---|---|
| SEQ ID NO: 1* | KSVWSKLQSIGIRQH | 51 +/-28 (n=7) |
| SEQ ID NO: 2* | LCYSILKAKNAGMS | Not done |
| SEQ ID NO: 4 | CKSVWSKLQSIGIRQH | 124 +/-31 (n=8) |
| SEQ ID NO: 5 | MKSVWSKLQSIGIRQH | 43 +/-14 (n=4) |
| SEQ ID NO: 6 | KKSVWSKLQSIGIRQH | 43 +/-29 (n=11) |
| SEQ ID NO: 7 | SKSVWSKLQSIGIRQH | 90 +/ 20 (n=7) |

| | | |
|---|---|---|
| * Described in Godet et al. Clin Can Res 2012 & Dosset et al Clin Can Res 2012. | | |

### Example 1. Binding of peptides on melanin

L-Dopa alone (0.8mg/ml) was polymerized into melanin for 2h at pH 8.5 and 60°C under agitation. The reaction mixture was then filtered on a 10kDa filter, and the retentate containing the synthetic melanin was resuspended at pH 7.5 in phosphate buffer. Peptides were then added (at the weight ratio melanin/peptide of 2) and the mixtures were incubated for 18 hours at room temperature.

Tricine-SDS-PAGE analysis was performed as described in Carpentier; 2017 (op. cit.). Briefly, samples (peptide-Mel or peptide alone) were loaded on acrylamide gels. Following electrophoresis, the gels were stained with Coomassie Brilliant Blue R-250 and imaged with the ChemiDoc XRS+ system (Bio-Rad Laboratory), allowing the quantification of the free peptide in the gel. The binding of peptides to melanin was expressed as the ratio: [amount of unbound peptide in samples Peptide-Mel / amount of peptides in control samples containing peptides alone]. As shown in table 3, binding was observed with SEQ ID NO: 7, which was unexpectedly much higher than the binding observed with the unmodified epitope peptide SEQ ID NO: 1, and higher than the binding observed with peptides modified according to the preferred embodiments of WO2021165306 (SEQ ID NO: 5 and SEQ ID NO: 6 for example).

**Table 3: Binding of peptides on synthetic melanin. L-Dopa (0.8mg/ml) underwent an oxidative polymerization at pH 8.5 in aerobic conditions for 2 hours at 60°C. The reaction mixture was then filtered on a 10kDa filter, and the retentate containing the synthetic melanin was resuspended at pH 7.5. Peptides were then added (at the weight ratio melanin/peptide of 2) and the mixture was incubated for various times at room temperature or 60°C. The percentage of peptides that bound to melanin was then quantified with SPS-page analysis**

| | 18hours, 20° C | 3 weeks 20° C | 1hour 60°C |
|---|---|---|---|
| SEQ ID NO: 1 | 41% | 64% | 55% |
| SEQ ID NO: 4 | 59% | / | / |
| SEQ ID NO: 5 | 50% | / | 55% |
| SEQ ID NO: 6 | 43% | / | 54% |
| SEQ ID NO: 7 | 60% | 90% | 65% |

### Example 2. Immunogenicity of the different peptides

The immunogenicity of these various peptides were screened by vaccinations in HLA-A2/DR1 mice (also known as Surel1 model). HLA-A2/DR1 mice are transgenic mice mimicking the human's immune system.

For immunizations, L-Dopa (0.8mg/ml) underwent an oxidative polymerization at pH 8.5 in aerobic conditions for 2 hours at 60°C. The reaction mixture was then filtered on a 10kDa filter, and the retentate containing the synthetic melanin was resuspended in water. Peptides (10 µg/mouse) were then added (at the weight ratio peptide/L-Dopa =1/4) and the mixtures were used for subcutaneous immunizations in mice after a 18-hour incubation time. Phosphorothioate oligonucleotide CpG-28 (5'-TAAACGTTATAACGTTATGACGTCAT, SEQ ID NO: 3) was added to vaccine formulations (10 µg/mouse) just before the immunizations. Mice were sacrificed on day 8 and the T-cell response was performed as described in Carpentier, 2017. Briefly, splenocytes were re-stimulated in vitro with the corresponding peptide (non-conjugated to melanin) and the numbers of IFNg-SFCs (Spot forming cells) were measured and expressed as Mean+/-S.E.M.

Surprisingly and unexpectedly, it was observed that peptide SEQ ID NO: 7 was very efficient to trigger an immune response after immunization in mice (Table 2). Namely, SEQ ID NO: 7 was more efficient than most preferred modifications recommended in WO2021165306 (methionine or lysine at the NH2-terminal depicted as SEQ ID NO: 5 and SEQ ID NO: 6), and nearly as efficient as SEQ ID NO: 4 that carries a cysteine at the NH2-terminal.

### Example 3. Peptide stability and filterability in solution

To develop a relevant vaccine, it is necessary to have a proper drug substance stability. To assess stability, the peptides were diluted at à 0,5 mg/mL in water, adjusted at pH 7.4, frozen at -20° C, and analyzed by HPLC (Hypersil GOLD C8 15cm x 4.6mm x 5µm (ThermoFisher) on the indicated day, using an elution buffer CH3CN 15%, TFA 0,1%. The pic area was compared to a reference standard.

As shown in table 4, SEQ ID NO: 5 and SEQ ID NO: 4 were highly unstable in solution at physiological pH (pH 7.4), while SEQ ID NO: 7 was very stable. Indeed, after 7 days at -20°C, the pic of SEQ ID NO: 4 was reduced by 53%, while SEQ ID NO: 7 was stable (> 95%) for at least 3 months at pH7.4 at -20°C, and at least 7 days at room temperature.

**Table 4: Stability of peptides after a 7-day incubation period in solution at pH7.4 at different temperature (ratio of the pick area on the indicated day/ pic area of a fresh solution of the same peptide)**

| | - 20°C | 4°C | Room temperature |
|---|---|---|---|
| SEQ ID NO: 1 | 100% | 98% | 92% |
| SEQ ID NO: 4 | 47% | Not done | Not done |
| SEQ ID NO: 5 | 23% | 25% | 3% |
| SEQ ID NO: 7 | 100% | 100% | 100% |

Filterability of a medical formulation on a 0.2µ filter is important to achieve a sterile solution. Surprisingly, SEQ ID NO: 7 appears particularly relevant for further pharmaceutical development as the mixture peptide + melanine, as described in example 2, does not precipitate and can be filtered on a 0.2µ filter, and remains in suspension for more than 18 hours in isotonic saline, while SEQ ID NO: 6 precipitates in such conditions.

### Example 4. Verification of the cross-reactivity of the selected peptide

SEQ ID NO: 7 thus presented good binding, immunogenicity, stability and filterability.

As SEQ ID NO: 7 is different from the natural epitope SEQ ID NO: 1, one can expect that this modification may result in a totally new epitope, having no cross reactivity with the native epitope. Mice were thus immunized with SEQ ID NO: 7, and it was checked whether T-cell response triggered by SEQ ID NO: 7 was able to similarly recognize the native epitope SEQ ID NO: 1.

It was shown that this is the case (Figure 1), thus demonstrating cross reactivity of the immune response on both epitopes.

### REFERENCES

Adotevi et al. J Clin Oncol. 2023 Jan 10;41(2):373-384. doi: 10.1200/JCO.22.00096. Epub 2022 Sep 7.
Armstrong et al. Mechanisms of Development 97, n° 1-2 (2000): 109-16. doi.org/10.1016/s0925-4773(00)00423-8.
Carpentier et al. PLoS One. 2017 Jul 17;12(7):e0181403. doi: 10.1371/journal.pone.0181403. eCollection 2017.
Chiodi, Ilaria, et Chiara Mondello. « Telomere-independent functions of telomerase in nuclei, cytoplasm, and mitochondria ». Frontiers in Oncology 2 (2012): 133. DOI: 10.3389/fonc.2012.00133.
Colebatch et al. Journal of Clinical Pathology 72, n° 4 (1 avril 2019): 281. doi: 10.1136/jclinpath-2018-205653.
Dogan and Forsyth. Cancers 13, no 6 (2021): 1213. doi: 10.3390/cancers13061213. Dosset et al Cancers (Basel). 2020 Jun 25;12(6):1687. doi: 10.3390/cancers 12061687
Dosset et al. Clin Cancer Res. 2012 Nov 15;18(22):6284-95. doi: 10.1158/1078-0432.CCR-12-0896
Dosset et al. Oncoimmunology. 2013 Mar 1;2(3):e23430. doi: 10.4161/onci.23430 Ellingsen et al. Front Immunol. 2021 Jul 5;12:682492. doi: 10.3389/fimmu.2021.682492.
Ghosh et al. Nature Cell Biology 14, n° 12 (2012): 1270-81. doi.org/10.1 038/ncb2621 Godet et al. Clin Cancer Res. 2012 May 15;18(10):2943-53. doi: 10.1158/1078-0432.CCR-11-3185
Gupta et al. The Journal of Molecular Diagnostics 23, n° 2 (2021): 253-63. Doi: 10.1016/j.jmoldx.2020.11.003
Hayflick L. Exp Gerontol. 1998 Nov-Dec;33(7-8):639-53. doi: 10.1016/s0531-5565(98)00023-0
Hiyama Br J Cancer. 2007 Apr 10;96(7):1020-4. doi: 10.1038/sj.bjc.6603671 Huang et al. Eur Urol. 2014 Feb;65(2):274-7. doi: 10.1016/j.eururo.2013.10.038 Killela et aL. Proc Natl Acad Sci U S A (2013) 110(15):6021-6. doi: 10.1073/pnas. 1303607110
Lee et al. J Clin Invest. 2019 Jan 2;129(1):223-229. doi: 10.1172/JCI121303
Li and Tergaonkar. Cancer Res. 2014 Mar 15;74(6):1639-44. doi: 10.1158/0008-5472.CAN-13-3568
Liu et al. 2016 Sci Rep. 2016 Mar 14;6:22886. doi: 10.1038/srep22886
Low, Kee Chung, et Vinay Tergaonkar. « Telomerase: Central Regulator of All of the Hallmarks of Cancer ». Trends in Biochemical Sciences 38, n° 9 (2013): 426-34. doi: 10.1016/j.tibs.2013.07.001
Middleton, G. et al. Lancet Oncol. 15, 829-840 (2014)
Palumbo et al, Biochim Biophys Acta. 1987; 13;925(2):203-9
Palumbo et al, Biochim Biophys Acta. 1991;1115(1):1-5
Pierini et al. Acta Neuropathol Commun. 2020 Aug 25;8(1):145. doi: 10.1186/s40478-020-01022-4.
Rosen et al. Redox Biology 34 (2020): 101543. DOI: 10.1016/j.redox.2020.101543 Slusher et al. Cancers 12, no 6 (10 juin 2020): 1514. doi: 10.3390/cancers12061514 Vonderheide, Biochimie 90 (2008) 173e180.
Wong et al. Trends in Genetics: TIG 30, n° 10 (2014): 430-38. doi.org/10.1016/j.tig.2014.07.006
Zhao et al. Cell 138, n° 3 (2009): 463-75. doi.org/10.1016/j.cell.2009.05.026

## Claims

1. A peptide comprising SEQ ID NO: 7.

2. The peptide of claim 1, which consists in SEQ ID NO: 7.

3. The peptide of claim 1, which comprises at most 50 amino acids.

4. The peptide of any one of claims 1 or 3, which further comprises other immunogenic epitopes.

5. A nucleic acid molecule coding for the peptide of any one of claims 1 to 4.

6. A vaccine composition comprising the peptide of any one of claim 1 to 4 or the nucleic acid molecule of claim 5.

7. The vaccine composition of claim 6, comprising the peptide of any one of claim 1 to 4 and an adjuvant.

8. The vaccine composition of claim 7, wherein the adjuvant is melanin.

9. The peptide of any one of claim 1 to 4, the nucleic acid of claim 5 or the vaccine composition of any one of claim 6 to 8 for use as a medicament or as a vaccine.

10. The peptide of any one of claim 1 to 7, the nucleic acid of claim 5 or the vaccine composition of any one of claim 6 to 8 for use for the treatment of cancer.

11. The peptide of any one of claim 1 to 4, the nucleic acid of claim 5 or the vaccine composition of any one of claim 6 to 8 for use according to claim 10, wherein the cancer is selected from the group consisting of glioblastomas, gliomas, melanomas, hepatocellular carcinoma, non small cell lung carcinomas, small cell lung carcinomas, urothelial and thyroid cancers.

12. The peptide of any one of claim 1 to 4, the nucleic acid of claim 5 or the vaccine composition of any one of claim 6 to 8 for use according to claim 10, wherein the cancer is a low- or high-grade glial tumor.

13. The peptide of any one of claim 1 to 4, the nucleic acid of claim 5 or the vaccine composition of any one of claim 6 to 8 for use according to any one of claims 9 to 12, which is in a form suitable for intramuscular, intravenous, subcutaneous, intraperitoneal, intratumoral oral, inhalable or intradermal administration.

14. An *in vitro* method for detecting, priming or stimulating lymphocytes directed against TERT, comprising contacting a peptide according to any one of claims 1 to 4, or a nucleic acid molecule according to claim 5, optionally with an adjuvant, with antigen presenting cells and T-lymphocytes.
